# FASCICULE DE BREVET EUROPEEN

(11) **EP 1 531 756 B1**
(45) Date de publication et mention de la délivrance du brevet: **14.05.2008**
(21) Numéro de dépôt: 03758281.4
(22) Date de dépôt: 27.08.2003
(51) Int. Cl.: A61F 2/00

(54) **DISPOSITIF FORMANT PROTHESE CHIRURGICALE, POUR L'IMPLANTATION D'UN SOUTENEMENT D'UN ORGANE D'UN MAMMIFERE**
CHIRURGISCHE PROTHESE ZUM STÜTZEN EINES ORGANS
SURGICAL PROSTHESIS-FORMING DEVICE USED TO IMPLANT AN ORGAN SUPPORT IN A MAMMAL

(30) Priorité: 30.08.2002 FR 0210761
(43) Date de publication de la demande: 25.05.2005
(73) Titulaire: Bouffier, Bernard, 25480 Ecole (FR)
(72) Inventeur: Bouffier, Bernard, 25480 Ecole (FR)
(74) Mandataire: Portal, Gérard
(86) Numéro de dépôt international: PCT/FR2003/002589
(87) Numéro de publication internationale: WO 2004/019813

(56) Documents cités:
- EP-A- 1 159 920
- WO-A-02/30293
- WO-A-94/26215
- US-A- 5 713 829

## Description

La présente invention concerne essentiellement un dispositif chirurgical formant prothèse chirurgicale, pour réaliser l'implantation d'un soutènement d'un organe d'un mammifère. Dans le cadre de l'invention, tout organe d'un mammifère, pour lequel le soutènement peut être réalisé, peut être traité par le dispositif chirurgical selon l'invention. L'invention est particulièrement adaptée au soutènement des organes pelviens, de l'urètre, de la vessie, du vagin, du col de l'utérus, de l'utérus et du rectum. De préférence, l'invention concerne un dispositif chirurgical formant prothèse chirurgicale pour réaliser un soutènement urétral pour le traitement de l'incontinence urinaire, en particulier d'un mammifère femelle, de préférence une femme.

Ainsi, le dispositif formant prothèse chirurgicale selon la présente invention est prévu pour fixer ou maintenir des tissus ou des organes différents, en apportant éventuellement des modifications plus significatives, imposées par les conditions anatomiques ou pathologiques.

### ETAT DE LA TECHNIQUE

On sait que l'incontinence urinaire d'efforts est la conséquence d'une mobilité excessive de l'urètre lors d'une pression abdominale importante, provoquée par la toux, le rire, la course, plus généralement une poussée abdominale.

Les fuites d'urine surviennent d'autant plus facilement et abondamment que la mobilité urétrale est importante, voire que le canal est ptosé en permanence.

L'incontinence peut être associée à une prolapsus des organes pelviens (vagin, utérus, vessie, rectum), dans des proportions variables. Elle peut être isolée. Elle est presque toujours la conséquence plus ou moins tardive des accouchements par voie basse.

Elle réalise une redoutable infirmité, atteignant douloureuse à la liberté et à la dignité des mamans ; elle est volontiers tenue secrète, ce qui rend aléatoire les études de morbidité.

Par contre, et c'est important, il ne s'agit pas d'une maladie mortelle, ce qui apparente sa prise en charge médicale à un traitement fonctionnel, avec le devoir pour le médecin de rechercher un résultat parfait au prix d'une agression minimale et de complication inexistante.

Une première avancée significative a été réalisée avec le dispositif dit TVT (Tension free Vaginal Tape), qui reprend le principe ancien d'une bandelette de soutien sous l'urètre en innovant par l'utilisation d'une bandelette de polypropylène, matériau très connu en particulier pour les prothèses herniaires, et par une mise en place mini-invasive.

Le document EP-A-0 248 544 décrit un dispositif formant prothèse chirurgical permettant le traitement de l'incontinence féminine, comprenant un corps composé d'un hydrogel polymère synthétique réticulé, de préférence avec un renforcement local, tel qu'un filet en matériau plastique, par exemple en polypropylène (voir colonne 3, lignes 39 à 46).

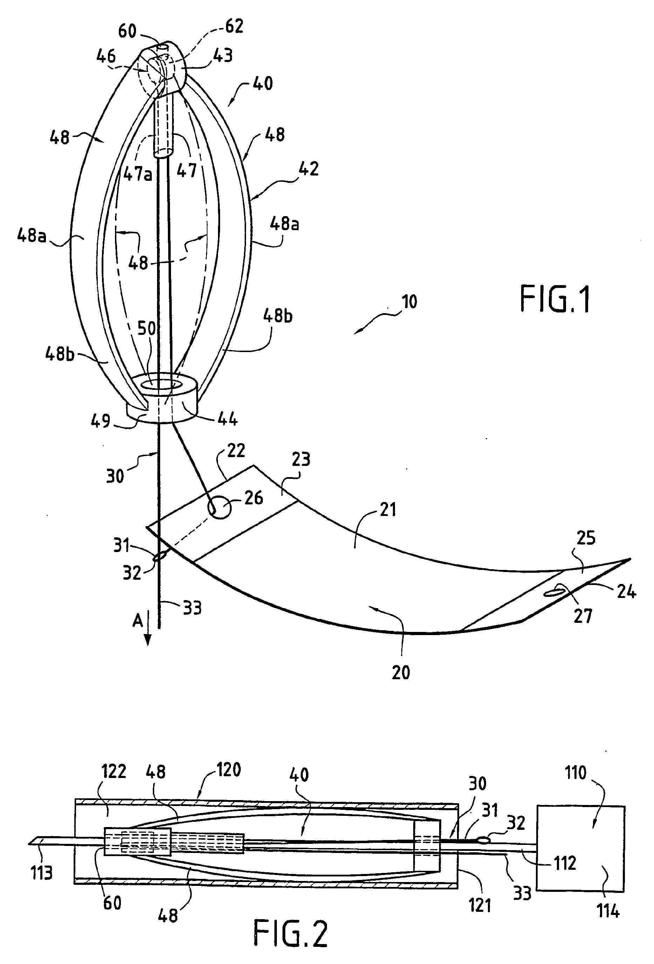

Le corps est suspendu par des sutures aux tissus para-urétrains pelviens, ou à l'aide d'une longue aiguille à la paroi abdominale antérieure (colonne 4, lignes 1 à 19 et figures 2 à 4).

Il est aussi connu par les documents US 5 899 909 et WO 02/30293, tel que décrit dans le préambule de la revendication 1 un appareil et un procédé de traitement de l'incontinence urinaire de la femme, et en particulier un implant chirurgical comprenant une bandelette destinée à venir se positionner sous l'urètre pour supporter l'urètre pour éviter l'incontinence, cette bandelette coopérant avec deux fils respectivement solidaires de chaque extrémité de la bandelette, reliés à l'opposé de la bandelette à un dispositif d'ancrage, en forme d'aiguille, et venant traverser des tissus de la paroi abdominale ou de la paroi pelvienne (« rectus sheath »).

La demande WO 98/35632 est relatif à d'autres modes de réalisation de bandelettes de tension avec renforcement aux extrémités réalisés par un repli des bords de la bandelette sur elle-même en formant ainsi une double épaisseur. La fixation peut avoir lieu sur l'os pelvien.

Les résultats fonctionnels sont excellents, mais la mise en place de la prothèse reste agressive de par la taille des deux aiguilles et du trajet qu'elles empruntent.

Une conséquence devenue inacceptable consiste en de nombreuses complications et des décès à déplorer de sorte que ce procédé est très médiatisé, notamment en France, mais ne se généralise pas vraiment dans de nombreux pays. En pratique, le protocole n'est pas facilement reproductible et la courbe d'apprentissage des praticiens n'est pas si rapide qu'on pouvait l'imaginer.

### BUTS DE L'INVENTION

La présente invention a pour but principal de résoudre le nouveau problème technique consistant en la fourniture d'un dispositif chirurgical formant prothèse chirurgicale de fixation et de soutien d'un organe biologique, notamment pour le soutènement urétral pour le traitement de l'incontinence urinaire, en particulier d'un mammifère femelle, de préférence une femme, qui soit moins agressif que les dispositifs antérieurs, et qui permette ainsi de limiter ou quasi-supprimer le risque de complications graves.

La présente invention a aussi pour but principal de résoudre le nouveau problème technique consistant en la fourniture d'un dispositif chirurgical formant prothèse chirurgicale de fixation et de soutien de tout organe d'un mammifère, pour lequel le soutènement peut être réalisé, qui soit avantageusement particulièrement adaptée au soutènement des organes pelviens, de l'urètre, de la vessie, du vagin, du col de l'utérus, de l'utérus et du rectum.

La présente invention a encore pour but principal de résoudre ce nouveau problème technique selon un dispositif qui permette la mise en oeuvre d'un procédé chirurgical de fixation et de soutien selon une procédure d'intervention rapide, sous anesthésie locale, sans hospitalisation, et sans immobilisation post-opératoire, donc réalisable « en ambulatoire ».

L'invention permet pour la première fois de résoudre ces problèmes techniques d'une manière simple, fiable, reproductible à l'échelle industrielle et médicale, en facilitant la procédure chirurgicale en permettant une intervention rapide, sous anesthésie locale, sans hospitalisation, et sans immobilisation post-opératoire.

Ainsi, selon un premier aspect, la présente invention fournit un dispositif chirurgical formant prothèse chirurgicale, destiné au soutènement d'un organe biologique à soutenir d'un mammifère, notamment à traiter l'incontinence urinaire, comprenant :
a) au moins un premier élément de soutènement comprenant un élément de forme allongée définissant une première et une deuxième extrémités pour exercer une action de soutènement dudit organe biologique ; ledit premier élément de forme allongée étant réalisé au moins en partie en un matériau sensiblement non extensible, souple, déformable ;
b) au moins un premier élément de traction, avantageusement filiforme, pouvant être rendu solidaire au moins provisoirement avec au moins une extrémité dudit premier élément de soutènement allongé, réalisé en un matériau sensiblement non extensible et permettant la traction et le maintien du premier élément de soutènement allongé, dans une position adaptée pour réaliser ledit soutien ;
c) au moins un premier système de fixation ou d'ancrage, destiné à coopérer avec au moins ledit premier élément de traction, non extensible, de manière à permettre la fixation et le maintien en place en position appropriée dudit premier élément de soutènement allongé. Le système est

caractérisé par ledit premier système d'ancrage comprenant un dispositif formant cage comportant une extrémité supérieure et une extrémité inférieure, l'extrémité supérieure étant pourvue d'un élément de glissement facilitant la traction de l'élément de traction agissant sur l'élément de soutènement allongé, afin d'exercer par ladite traction un mouvement de translation d'une extrémité de l'élément de soutènement allongé pour réaliser le soutènement et/ou le maintien dudit organe.

Selon un mode de réalisation avantageux de l'invention, ladite extrémité inférieure du système d'ancrage est conformée pour prendre appui ou se fixer sur un support relativement résistant au déchirement du mammifère, tel que os ou tissus appropriés. Dans le cas de la réalisation d'un soutènement pour traiter l'incontinence urinaire, il est préféré que l'extrémité inférieure du système d'ancrage vienne prendre appui sur l'os pubien ou des tissus de la paroi pubienne ou pelvienne ou éventuellement abdominale.

Selon encore un mode de réalisation avantageux de l'invention, la partie supérieure et la partie inférieure de l'élément formant cage du système d'ancrage sont reliées entre elles par plusieurs bras de liaisons latéraux réalisés en un matériau sensiblement non extensible, mais souple, déformable.

Avantageusement, lesdits bras de liaisons latéraux sont d'une longueur suffisante pour former sensiblement en leur milieu un point de pliage desdits bras latéraux, de manière que la partie supérieure sera replié en direction de la partie inférieure, la partie des bras solidaire de la partie inférieure venant alors prendre appui également sur le support précité relativement résistant au déchirement du mammifère, tel que os ou tissus appropriés. Ainsi, le système d'ancrage prend appui sur le support relativement résistant au déchirement du mammifère sur une grande surface, ce qui limite ou empêche son déchirement, ce qui est particulièrement avantageux dans le cas où le support d'appui comprend des tissus du mammifère, et en particulier la paroi pubienne ou pelvienne ou éventuellement abdominale.

Selon un mode de réalisation avantageux de l'invention, l'élément de glissement du système d'ancrage en forme de cage est conçu pour agir comme une poulie, autour de laquelle vient glisser ou coulisser l'élément de traction afin d'exercer l'effet de traction sur l'élément de soutènement allongé.

Selon un mode de réalisation particulièrement avantageux de l'invention, indépendamment brevetable, l'élément de traction se présente sous la forme d'un fil de traction, de préférence comportant à une extrémité un élément de coulissement, tel qu'un anneau ou une boucle, dans laquelle peut être insérée l'autre extrémité ou partie libre du fil, de manière à permettre la formation d'un dispositif en boucle type lasso permettant une traction aisée sur l'élément de soutènement allongé. Dans ce cas, l'élément de soutènement allongé présente de préférence au moins un orifice dans lequel peut passer ledit fil de traction pour être ainsi prisonnier du dispositif type lasso, qui, par une traction réalisée sur l'autre extrémité ou la partie libre du fil réalise la translation par traction de ladite extrémité de l'élément de soutènement allongé, par exemple une bandelette.

Selon un autre mode de réalisation particulièrement avantageux de l'invention, il peut être prévu que chaque extrémité de l'élément de soutènement allongé, par exemple une bandelette, peut être tractée simultanément par un système d'ancrage unique.

D'autre part, il peut être prévu un premier système d'ancrage solidaire d'une première extrémité de l'élément de soutènement allongé, par exemple une bandelette, pour permettre une fixation à une position prédéterminée fixe, tandis que l'autre extrémité de l'élément de soutènement allongé, par exemple une bandelette, est rendue solidaire d'un second système d'ancrage selon la présente invention permettant d'exercer un déplacement de la deuxième extrémité, ici encore mobile, de l'élément de soutènement allongé, pour exercer l'effet de traction recherché sur l'organe du mammifère en ayant besoin.

Selon un autre mode de réalisation de l'invention, le dispositif formant cage précité comprend à son extrémité supérieure un élément tubulaire creux dont une extrémité est conçue pour être à la fois solidarisable à, ou monobloc avec, la partie supérieure de la cage et former l'élément de glissement, en particulier en présentant une surface formant poulie autour de laquelle le fil de traction est destiné à glisser ; avantageusement, ladite extrémité comprend un orifice traversant coaxial avec l'élément tubulaire creux.

Selon encore un autre mode de réalisation de l'invention, la partie inférieure d'au moins le premier système d'ancrage ou de chaque système d'ancrage, peut comprendre une partie annulaire définissant un orifice central de passage du premier élément de traction, avantageusement filiforme précité, ou de chaque élément de traction. Cette partie annulaire sert avantageusement aussi au passage de l'aiguille d'un trocart de pénétration dans les tissus.

Egalement, selon un mode de réalisation avantageux, la partie supérieure d'au moins un premier système d'ancrage ou de chaque système d'ancrage, peut comprendre un orifice traversant permettant aussi le passage de l'aiguille d'un trocart de pénétration dans les tissus. Ainsi, il est possible de mettre en place de manière sûre et fiable chaque système d'ancrage à l'intérieur d'un trocart de pénétration entre une aiguille de pénétration du trocart et une gaine extérieure, selon une direction sensiblement axiale du système d'ancrage, et comme cela est clairement compréhensible pour l'homme de l'art à partir notamment de la figure 2 annexée, qui sera décrite plus loin.

Selon un deuxième aspect, la présente invention couvre également en tant que produit nouveau indépendamment brevetable l'élément de traction lui-même tel que précédemment défini ou tel que résultant de la description suivante, faite en référence aux dessins, qui font partie intégrante de la présente invention et complètent la description.

Selon une variante de réalisation particulière de l'invention, le premier élément de soutènement allongé présente la forme d'une bandelette, dont au moins la partie entre ses extrémités est réalisée sous la forme d'un tissu ou treillis formé à partir d'un ou de plusieurs fils réalisés en matériau souple, déformable, mais sensiblement non extensible.

Avantageusement, le matériau utilisé pour la fabrication du ou des fils servant à former le tissu de la bandelette est un polymère organique compatible avec une implantation à l'intérieur des tissus d'un mammifère, ce matériau organique étant avantageusement choisi parmi le polyéthylène, le propylène, ou le nylon, de préférence le polypropylène.

Selon une autre variante de réalisation de l'invention, le premier et/ou le deuxième élément de traction, filiforme, comprend ou est constitué d'un fil de traction non extensible, en particulier d'un fil réalisé en un matériau non extensible, par exemple un polymère organique compatible avec une implantation permanente dans les tissus d'un mammifère, en particulière le polyéthylène, le polypropylène ou le nylon.

Selon un troisième aspect, la présente invention concerne aussi en tant que produit nouveau indépendamment brevetable chaque système de fixation ou d'ancrage tel que précédemment défini ou tel que résultant de la description suivante faite en référence aux dessins, qui font partie intégrante de la présente invention, et qui complètent donc la présente description.

Selon un quatrième aspect, la présente invention couvre encore un kit de fixation et d'ancrage caractérisé en ce qu'il comprend au moins un dispositif chirurgical formant prothèse chirurgicale, selon l'un quelconque des aspects précédents, ainsi qu'un dispositif introducteur avantageusement sous forme d'un trocart de pénétration, avec une gaine de protection du système de fixation ou d'ancrage monté de manière compacte ou repliée entre le trocart de pénétration et la gaine et est solidaire de ladite gaine au moins dans le sens de la pénétration, ladite gaine comportant un élément de blocage anti-retrait du système de fixation en place entre le trocart et la gaine.

La méthode chirurgicale pour réaliser le soutènement d'un organe à soutenir d'un mammifère en ayant besoin, comprend les étapes suivantes :
a) une anesthésie locale au voisinage de l'organe à soutenir ;
b) une incision et une dissection des tissus en regard et de part et d'autre de l'organe à soutenir dudit mammifère ;
c) mise en place du dispositif introducteur dans le passage créé par ladite incision et dissection des tissus, sur un côté et au-delà de l'organe à soutenir, et après franchissement du support relativement résistant au déchirement dudit mammifère, tels que os ou tissus appropriés, retrait de la gaine de protection,
d) retrait du trocart de pénétration, notamment de sa partie formant aiguille ;
e) traction simultanée sur les deux extrémités de l'élément de traction, avantageusement filiforme, permettant le positionnement et l'ouverture, ou déploiement du système de fixation ou d'ancrage, et avantageusement dans le mode de réalisation préférée son pliage en au moins deux parties des bras de liaison pour former un positionnement dit en parapluie présentant une grande surface de fixation ou d'ancrage du système d'ancrage ;
f) répétition des étapes précédentes c), d) et e) de l'autre côté de l'organe pour introduire un deuxième système de fixation, de préférence identique au premier ;
g) passage d'une extrémité de l'élément de traction du premier système de fixation dans un moyen de solidarisation, tel qu'un orifice d'une première extrémité du premier élément de soutènement allongé, puis dans l'élément de coulissement, tel qu'anneau ou boucle de l'autre extrémité de l'élément de traction filiforme, de manière à former le dispositif en boucle type lasso et réalisation d'un premier mouvement de traction sur l'extrémité ou partie libre de l'élément de traction pour un pré-positionnement ;
h) réalisation de la même procédure qu'à l'étape précédente concernant le deuxième système de fixation de l'autre côté de l'organe à soutenir en obtenant ainsi un pré-positionnement de l'élément de soutènement de forme allongée près de l'organe à soutenir ;
i) action simultanée sur les deux extrémités ou partie libre respectivement du premier élément de traction et du deuxième élément de traction jusqu'à mise en tension du premier élément de soutènement allongé en position appropriée en contact avec ledit organe pour réaliser le soutien recherché dudit organe ;
j) solidarisation de l'extrémité libre respectivement du premier élément filiforme et de l'extrémité du deuxième élément filiforme dans la position optimale, cette solidarisation desdites extrémités étant réalisée à une position appropriée pour ne pas créer une gène vis-à-vis de l'organe ainsi soutenu ;
k) fermeture de l'incision avec un moyen de fermeture chirurgicale appropriée, tel que fil résorbable.

Ce procédé chirurgical, est utilisé pour réaliser le traitement de l'incontinence d'un mammifère femelle, en particulier d'une femme. Dans ce cadre, l'organe à soutenir est l'urètre de préférence au voisinage de la partie urétrale proche de la vessie. Dans ce cadre, il est préféré que les systèmes d'ancrage prennent appui sur les tissus de la paroi pelvienne, de préférence des tissus sensiblement transversaux de la paroi pelvienne dans la partie située entre l'urètre et les côtés du mammifère femelle, en particulier une femme, au-dessus de la paroi vaginale.

Dans les dessins :
- la figure 1 représente de manière éclatée en élévation, les trois éléments principaux du dispositif chirurgical formant prothèse chirurgicale comprenant au moins un premier élément de soutènement de forme allongée, par exemple une bandelette ; au moins un premier système de fixation ou d'ancrage comprenant un élément formant cage ; et au moins un premier élément de traction avantageusement filiforme, de préférence formant un dispositif en boucle type lasso, comme cela est bien visible à la figure 1, deux bras de liaison (48) sont représentés en traits mixtes ;
- la figure 2 représente le premier système de fixation ou d'ancrage, équipé d'au moins un premier élément de traction, avantageusement filiforme, en place sur un trocart de pénétration, pourvu d'une gaine extérieure de protection pour la mise en place chirurgicale ;
- la figure 3 représente une vue schématique du système de fixation ou d'ancrage déployé après retrait de la gaine de protection et du trocart de pénétration, et première action sur les deux extrémités de l'élément de traction filiforme pour plier le système d'ancrage avec appui sur le support relativement résistant au déchirement, tels que os ou tissus appropriés ;
- la figure 4 représente une vue en coupe selon la ligne de coupe IV-IV de la figure 3 ; et
- la figure 5 représente selon l'application chirurgicale actuellement préférée le soutien d'un organe d'un mammifère, ici pour le traitement de l'incontinence féminine avec de ce fait soutien de l'urètre.

En référence à la figure 1, on a représenté par le numéro de référence général (10) un dispositif chirurgical formant prothèse chirurgicale selon la présente invention. Celui-ci est adapté pour le soutènement d'un organe biologique à soutenir d'un mammifère. Il est dans le mode de réalisation préféré représenté particulièrement destiné à traiter l'incontinence urinaire, en particulier l'incontinence urinaire d'un mammifère femelle, de préférence celle de la femme.

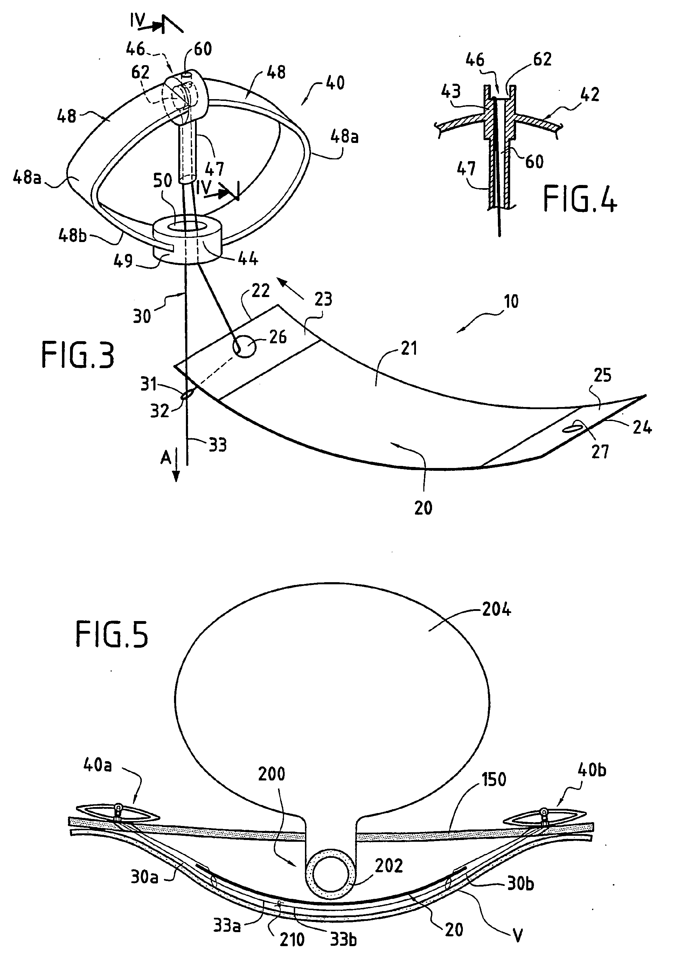

Ce dispositif (10) comprend :
a) au moins un premier élément de soutènement représenté par le numéro de référence général (20), de forme allongée, définissant une première extrémité (22) et une deuxième extrémité (24). Ce premier élément de soutènement (20) est conçu pour exercer une action de soutènement d'un organe biologique à soutenir d'un mammifère, tel que l'urètre, comme cela est représenté à la figure 5.
   Ce premier élément de soutènement (20) est réalisé en moins en partie en un matériau non extensible, souple, déformable. Il est de préférence totalement en ce matériau non extensible.
b) au moins un premier élément de traction (30), avantageusement filiforme, par exemple comprenant un fil, ou toron formé de la combinaison de plusieurs fils, pouvant être rendu solidaire au moins provisoirement avec au moins une extrémité (22) ou (24) dudit premier élément de soutènement allongé. Ce premier élément de traction (30) peut être réalisé en un matériau sensiblement non extensible et permettant la traction et le maintien du premier élément de soutènement allongé (20) dans une position adaptée pour réaliser ledit soutien, comme cela est bien compréhensible à partir de la description précédente et de la description suivante, qui sera faite en référence aux figures 3 à 5.
c) au moins un premier système de fixation ou d'ancrage représenté par le numéro de référence général (40), destiné à coopérer avec au moins ledit premier élément de traction (30), non extensible, de manière à permettre la fixation et le maintien en place en position appropriée dudit premier élément de soutènement (20). Le dispositif est

caractérisé par le système d'ancrage (40), qui comprend un élément formant cage représenté par le numéro de référence général (42) comportant une extrémité supérieure (43) et une extrémité inférieure (44), l'extrémité supérieure (43) étant pourvue d'un élément de glissement (46) facilitant la traction de l'élément de traction (30) agissant sur l'élément de soutènement allongé (20), afin d'exercer par ladite traction un mouvement de translation d'une extrémité (22) ou (24) de l'élément de soutènement allongé pour réaliser le soutien et ensuite le maintien dudit organe.

Selon le mode de réalisation actuellement préféré, tel que représenté, le premier élément de soutènement allongé (20) présente la forme d'une bandelette (21), dont au moins la partie entre ses extrémités est réalisée sous forme d'un tissu ou treillis formé à partir d'un ou plusieurs fils réalisé en un matériau souple, déformable, mais sensiblement non extensible. La réalisation de telles bandelettes est bien connue de l'homme de l'art et est décrite dans les documents précédemment cités de l'art antérieur, l'homme du métier pourra en particulier se reporte au document WO 98/35632, qui décrit diverses variantes de réalisation, avec des zones de renforcement, qui peuvent être obtenues par repliage des extrémités comme montré par exemple à la figure 6 (figures 6A à 6O), ou encore aux figures 7 à 9 de celui-ci.

Les dimensions de la bandelette sont aussi décrites en détail dans l'art antérieur, notamment dans le document WO 98/35632, de la page 7, ligne 22 à la page 15, ligne 33.

Dans le cadre de la présente invention, il est préféré que le matériau utilisé pour la fabrication du ou des fils servant à former le tissu de la bandelette soit un polymère organique compatible avec une implantation à l'intérieur des tissus d'un mammifère, ce matériau organique étant avantageusement choisi parmi le polyéthylène, le polypropylène, le nylon, de préférence le polypropylène.

Dans le cadre de l'invention, on peut avantageusement prévoir des extrémités (22), (24) comportant au moins une zone de renforcement telle que (23), (25) formée par un tissage différent des fils, soit avantageusement par un, ou de préférence au moins deux, pliages sur elle-même de la bandelette, de manière à fournir une double ou même une triple épaisseur. Ensuite, on peut prévoir la formation de moyens de solidarisation, au moins provisoire avec l'élément de traction (30), tel qu'un orifice respectivement (26) et (27) pour chaque extrémité (22), (24).

Dans le cadre de l'invention, selon un mode de réalisation avantageux, indépendamment brevetable, l'élément de traction (30) se présente sous la forme d'un fil de traction, de préférence comportant à une extrémité (31) un élément de coulissement (32), tel qu'un anneau ou une boucle, qui peut être formé(e) par un noeud sur lui-même du fil approprié, dans laquelle peut être insérée l'autre extrémité (33) ou partie libre du fil, de manière à former un dispositif en boucle type lasso, comme cela est bien compréhensible à un homme de l'art, en permettant ainsi une fraction aisée sur l'élément de soutènement allongé (20), comme cela expliqué plus loin. Dans ce cas, l'élément de soutènement présente, comme cela était décrit, au moins un orifice (26) ou (27) dans lequel peut passer le fil traction (30), de sorte que l'extrémité correspondante, ici (22), figure 1, du dispositif de soutènement (20) est ainsi prisonnière du dispositif type lasso. Ainsi, par une traction réalisée sur l'autre extrémité, ou partie libre (33), dans le sens de la flèche A indiquée, on peut réaliser la translation par une traction de l'extrémité (22) de l'élément de soutènement (20), par exemple ici une bandelette.

Le système d'ancrage (40), qui est lui est aussi indépendamment brevetable, sera maintenant décrit plus en détail. Celui-ci comprend un dispositif formant cage (42), qui comprend selon un mode de réalisation préféré représenté, à son extrémité supérieure (43), un élément de glissement (46) pouvant être formé par exemple par un élément tubulaire creux (47), dont une extrémité (47a) est conçue pour être à la fois solidarisable à, ou monobloc avec ( comme représenté), ladite partie supérieure (43) de la cage (42) et former ledit élément de glissement (46), en particulier en présentant une surface formant poulie de déplacement ou de glissement autour de laquelle est destiné à passer le fil de traction (30) précité, comme représenté à la figure 1. L'extrémité (47a) est ici monobloc avec l'extrémité supérieure (43) qui comporte aussi une gorge (62) formant surface de glissement de l'élément de glissement (46). L'extrémité (47a) comprend un orifice traversant (60) coaxial avec l'élément tubulaire creux (47), l'ensemble constituant ici un élément monobloc avec l'extrémité supérieure (43) de la cage (42).

Ainsi, l'orifice (60) de l'extrémité supérieure (43) du dispositif formant cage (42) est particulièrement destiné au passage de l'aiguille (113) du dispositif introducteur formant trocart décrit plus loin en référence à la figure 2. L'homme de l'art comprend aussi que l'orifice est coaxial avec l'axe de l'orifice traversant défini par l'élément tubulaire creux (47), pour permettre de servir de guide de passage de l'aiguille (113) du dispositif introducteur formant trocart, comme cela sera aussi décrit en référence à la figure 2.

Selon un mode de réalisation avantageux de l'invention, la partie supérieure (43) et la partie inférieure (44) de la cage (42) sont reliées entre elles par plusieurs bras de liaison latéraux (48), par exemple au nombre de quatre, à la figure 2, deux des bras (48) sont représentés en traits mixtes, réalisés en un matériau non extensible, biologiquement compatible, comme le reste de la cage et l'ensemble des éléments de la présente invention. Ce matériau est de préférence un matériau organique biocompatible, avantageusement choisi parmi le polyéthylène, le polypropylène, le nylon, et de préférence le polypropylène.

Avantageusement, la longueur des bras de liaison latéraux (48) est telle que ceux-ci peuvent se plier facilement lorsqu'il est exercé un effort de traction à l'aide de l'élément de traction (30), pliage qui est commencé dans la représentation schématique de la figure 1. Ce pliage se fait généralement au voisinage du milieu (48a) de la longueur des bras (48), comme cela est bien compréhensible à un homme de l'art. Ceci permettra, comme représenté aux figures 3 et 4, d'obtenir une déformation du dispositif d'ancrage de type parapluie, selon laquelle les parties inférieures des bras en dessous du point de pliage (48a) forment une grande surface d'appui du dispositif d'ancrage sur les tissus de support du dispositif d'ancrage, en diminuant ou même empêchant des effets de déchirement de ces tissus, comme cela sera aussi expliqué plus loin.

Selon le mode de réalisation préféré représenté, la partie inférieure (44) du dispositif d'ancrage (40) présente une forme annulaire (49) définissant un orifice central (50) au travers duquel peuvent passer les deux parties du fil en lasso. La forme annulaire n'est évidemment pas limitative et diverses formes sont possibles. Il est avantageux que la partie inférieure soit relativement solide pour former une embase solide à partir de laquelle le pliage des bras de liaison latéraux (48) en est facilité.

En référence à la figure 2, on a représenté le dispositif introducteur (100) qui comprend d'une part un trocart de pénétration (110) se composant d'une aiguille proprement dite (112) comportant une partie effilée distale (113) et une partie proximale (114) évasée formant poignée de poussée. Ce dispositif introducteur (100) comprend aussi une gaine extérieure (120), classique dans les systèmes de trocart introducteur, de diamètre suffisamment plus grand que le diamètre de l'aiguille (112), afin de réserver une chambre (122) formant logement pour le dispositif d'ancrage (40), à l'état replié, pré-équipé de l'élément de traction (30), avec sa première extrémité (31) comportant l'élément de coulissement (32), tel qu'anneau ou boucle, et la deuxième extrémité libre (33), les deux extrémités sortant à l'extérieur de la gaine étant d'une longueur suffisante pour être accessible par le chirurgien après introduction du dispositif introducteur jusqu'au lieu de libération déterminé par le praticien, généralement au-dessus d'un support, tel que par exemple la paroi pelvienne transversale (150) représentée à la figure 4. La partie arrière de la gaine peut avantageusement comporter un élément de compression (121), tel qu'une vis destinée à fixer la gaine à l'aiguille (112) pendant sa progression et à former un moyen anti-retrait du dispositif d'ancrage (40). Ce dispositif introducteur utilisant un trocart de pénétration et une gaine est classique pour un chirurgien. L'aiguille (112) étant avantageusement creuse et courbe et comporte à quelques centimètres de son extrémité distale un renflement ou butée (113a) destiné(e) à empêcher le recul du dispositif d'ancrage (40) positionné ou de l'aiguille (112) à l'intérieur de la gaine (120). Cette aiguille courte (112) comporte à son extrémité proximale le renflement où la poignée (114), qui est avantageusement destinée à recevoir par exemple une seringue pour l'injection du produit anesthésiant, le renflement permettant une bonne préhension de l'aiguille par l'opérateur pour lui permettre de la positionner avec précision, comme cela est bien connu aux chirurgiens.

En référence aux figures 3 et 4, on a représenté la fin de la première étape de la mise en place du dispositif d'ancrage (40), alors que celui-ci a été déployé par retrait tout d'abord de la gaine puis de l'aiguille (112). En outre, on a représenté schématiquement, l'extrémité (22) du dispositif de soutènement (20) avec l'élément de traction (30) dont une première extrémité du fil (31) a été insérée au travers de l'orifice (26), tandis que l'autre extrémité (33) du fil a été insérée dans l'élément de coulissement (32), tel qu'un anneau ou une boucle, de manière à former un dispositif en boucle de type lasso, comme bien visible à la figure 3, et comme déjà montré à la figure 1. En exerçant une traction simultanée sur les deux extrémités de l'élément de traction (30), on produit une ouverture dite en parapluie et un positionnement du système d'ancrage (40), avec un pliage sensiblement au niveau du milieu (48a) des bras de liaison (48) de sorte que la partie inférieure (48b) des bras (58) constitue en combinaison une grande surface de soutien et donc d'ancrage du système d'ancrage (40), sans sensiblement exercer un effet de cisaillement ou de déchirement des tissus qui le supportent. L'homme de l'art comprend que pour réaliser le support d'un organe à soutenir, tel que l'organe (200) représenté à la figure 5, ici selon l'application actuellement préférée, l'urètre (202), qui aboutit à la vessie (204), le praticien doit insérer avec le dispositif introducteur (100) deux systèmes de fixation respectivement référencés (40a) et (40b) de part et d'autre de l'organe à soutenir (200), tel qu'ici l'urètre (202).

Il doit aussi emprisonner chaque extrémité respectivement (22) ou (24) de l'élément de soutènement (20), telle qu'une bandelette (21), dans le système de lasso, comme représenté à la figure 5, et ensuite exercer un effort de traction, tout d'abord sur les deux extrémités de l'élément de traction respectivement (33a) et (33b) pour chaque système d'ancrage (40a), (40b), de manière à déployer et plier les systèmes de fixation (40a), (40b), ici en forme dite de parapluie, pour qu'il vienne reposer solidement sur les tissus support, ici la paroi pelvienne transversale (150), (voir figure 4), à l'opposé de part et d'autre de l'organe à soutenir (200), pour exercer correctement l'effort de soutien recherché et comme cela est bien compréhensible à un homme de l'art, notamment à partir de la connaissance de l'état de la technique antérieure, tel que représenté par les documents cités dans l'état de la technique antérieure en début de la présente description.

A la fin de la traction, le chirurgien réalise la traction de chaque extrémité (22), (24) de l'élément de soutènement (20), tel qu'une bandelette, et lorsque la traction de soutien est suffisante, le praticien noue les deux extrémités libres respectivement (33a), (33b) de l'élément de traction (30a), (30b), en formant par exemple un noeud solide (210), qui est de préférence positionné en dehors de la zone de soutien de l'organe (200), tel que l'urètre (202), comme représenté à la figure 5.

Grâce à l'invention, il est possible de réaliser une chirurgie ambulatoire avec une simple anesthésie locale.

Dans le cadre de l'application au traitement de l'incontinence d'un mammifère femelle, en particulier une femme, il suffit de préparer la patiente par une simple douche et toilette vaginale habituelles. Il n'est pas nécessaire de réaliser une prémédication ou perfusion. Une simple antibioprophylaxie per os peut être réalisée.

En position gynécologique, les cuisses fléchies entre 40 et 60°, le chirurgien met en oeuvre le procédé de chirurgie précédemment décrit. Cette procédure de chirurgie comprend une anesthésie locale avec environ 20 cm³ de xilocaïne à 0,5 % sur la muqueuse vaginale à 1 cm du méat, puis latéralement, et enfin au-delà de la paroi pelvienne pour réaliser une hydrodissection. Le chirurgien pratiquera ensuite une incision de 1 cm à 1cm du méat.

Il réalise ensuite une dissection para-urétrale au ciseau sur 3 cm jusqu'à la paroi pelvienne à droite et à gauche de l'urètre.

Il procède ensuite à la mise en place du dispositif introducteur d'une main sous contrôle digitale intravaginale de l'autre main. Par l'incision, en progressant latéralement dans le décollement, il exerce une petite pression pour franchir la paroi pelvienne (150), puis il réalise une avancée de 2 cm sans forcer.

Il réalise ensuite le retrait de la gaine (120), puis ensuite du trocart (110).

La traction simultanée sur les deux extrémités (31) et (33) de chaque élément de traction (30) réalise l'ouverture en parapluie du dispositif d'ancrage (40), puis après le passage de l'extrémité (31) dans l'orifice (26) du premier dispositif de soutènement (20) et formation de la boucle en lasso, comme représenté à la figure 3, la traction de l'extrémité (33) ou (33a) ou (33b), voir figures 3 et 4, permet de réaliser la traction et la mise en place appropriée de l'élément de soutènement allongé (20). Il est à noter que pour certaines chirurgies visant des organes d'une certaine dimension, pour le traitement de certaines pathologies, il peut être nécessaire d'utiliser plusieurs éléments de soutènement allongé (20), ou un élément de soutènement allongé de plus grande largeur, au choix du chirurgien.

Dans le cadre de l'invention, tout organe d'un mammifère, pour lequel le soutènement peut être réalisé, peut être traité par le dispositif chirurgical selon l'invention. L'invention est particulièrement adaptée au soutènement des organes pelviens, de l'urètre, de la vessie, du vagin, du col de l'utérus, de l'utérus et du rectum.

## Revendications

1. Dispositif chirurgical formant prothèse chirurgicale (10), destiné au soutènement d'un organe biologique à soutenir d'un mammifère, notamment à traiter l'incontinence urinaire, comprenant :
a) au moins un premier élément de soutènement (20), de forme allongée définissant une première (22) et une deuxième (24) extrémités pour exercer une action de soutènement d'un organe biologique (200) d'un mammifère à soutenir ; ledit premier élément de forme allongée étant réalisé au moins en partie en un matériau sensiblement non extensible, souple, déformable ;
b) au moins un premier élément de traction (30), avantageusement filiforme, pouvant être rendu solidaire au moins provisoirement avec au moins une extrémité (22) ou (24) dudit premier élément de soutènement (20) allongé, réalisé en un matériau sensiblement non extensible et permettant la traction et le maintien du premier élément de soutènement (20) allongé dans une position adaptée pour réaliser ledit soutien ;
c) au moins un premier système de fixation ou d'ancrage (40), destiné à coopérer avec au moins ledit premier élément de traction (30), non extensible, de manière à permettre la fixation et le maintien en place en position appropriée dudit premier élément de soutènement allongé (20) ;
**caractérisé en ce que**
ledit premier système d'ancrage (40) comprenant un dispositif formant cage (42) comportant une extrémité supérieure (43) et une extrémité inférieure (44), l'extrémité supérieure (43) étant pourvue d'un élément de glissement (46) facilitant la traction de l'élément de traction (30) agissant sur l'élément de soutènement allongé (20), afin d'exercer par ladite traction un mouvement de translation d'une extrémité de l'élément de soutènement allongé (20) pour réaliser le soutènement et/ou le maintien dudit organe (200).

2. Dispositif selon la revendication 1, **caractérisé en ce que** l'extrémité inférieure (44) du système d'ancrage (40) est conformée pour prendre appui ou se fixer sur un support (150) relativement résistant au déchirement du mammifère, tel que os ou tissus appropriés.

3. Dispositif selon la revendication 1 ou 2, **caractérisé en ce que** la partie supérieure (43) et la partie inférieure (44) de l'élément formant cage (42) du système d'ancrage (40) sont reliées entre elles par plusieurs bras de liaisons latéraux (48) réalisés en un matériau sensiblement non extensible, mais souple, déformable.

4. Dispositif selon la revendication 3, **caractérisé en ce que** lesdits bras de liaisons latéraux (48) sont d'une longueur suffisante pour former sensiblement en leur milieu (48a) un point de pliage desdits bras latéraux (48), de manière que la partie supérieure sera repliée en direction de la partie inférieure, la partie des bras (48b) solidaire de la partie inférieure venant alors prendre appui également sur le support précité (150) relativement résistant au déchirement des mammifères, tel que os ou tissus appropriés ; le système d'ancrage prenant ainsi appui sur le support relativement résistant au déchirement des mammifères sur une grande surface, ce qui limite ou empêche son déchirement.

5. Dispositif selon l'une des revendications 1 à 4, **caractérisé en ce que** l'élément de glissement (46) du système d'ancrage (40) en forme de cage est conçu pour agir comme une poulie, autour de laquelle vient coulisser l'élément de traction (30) afin d'exercer l'effet de traction sur l'élément de soutènement allongé (20).

6. Dispositif selon l'une des revendications précédentes, **caractérisé en ce que** l'élément de traction (30) se présente sous la forme d'un fil de traction, de préférence comportant à une extrémité (31), un élément de coulissement (32), tel qu'un anneau ou une boucle, dans laquelle peut être insérée l'autre extrémité ou partie libre (33) du fil, de manière à permettre la formation du dispositif en boucle type lasso permettant une traction aisée sur l'élément de soutènement allongé (20) ; de préférence, l'élément de soutènement allongé (20) présente au moins un orifice (26, 27) dans laquelle peut passer ledit fil de traction (30) pour être ainsi prisonnier du dispositif type lasso, qui, par une traction réalisée sur l'autre extrémité ou la partie libre (33) du fil réalise la translation par traction de ladite extrémité (22) ou (24) de l'élément de soutènement allongé (20), par exemple une bandelette.

7. Dispositif selon l'une des revendications précédentes, **caractérisé en ce qu'**il est prévu un système d'ancrage unique pour tracter simultanément chaque extrémité (22, 24) de l'élément de soutènement allongé (20), par exemple une bandelette.

8. Dispositif selon l'une des revendications précédentes, **caractérisé en ce qu'** un premier système d'ancrage (40a) solidaire d'une première extrémité (22) de l'élément de soutènement allongé (20), par exemple une bandelette, est prévu pour permettre une fixation à une position prédéterminée fixe, tandis que l'autre extrémité (24) de l'élément de soutènement allongé (20), par exemple une bandelette, est rendue solidaire d'un second système d'ancrage (40b) permettant d'exercer un déplacement de la deuxième extrémité (24), de l'élément de soutènement allongé (20) pour exercer l'effet de traction recherchée sur l'organe (200) du mammifère en ayant besoin.

9. Dispositif selon l'une des revendications précédentes, **caractérisé en ce que** le dispositif formant cage (42) comprend à son extrémité supérieure (43) un élément tubulaire creux (47), dont une extrémité (47a) est conçue pour être à la fois solidarisable à, ou monobloc avec, la partie supérieure (43) de la cage (42) et former l'élément de glissement (46), en particulier en présentant une surface formant poulie autour de laquelle le fil de traction (30) est destiné à glisser ; avantageusement, ladite extrémité (47a) comprend un orifice traversant (60) coaxial avec l'élément tubulaire creux (47).

10. Dispositif selon l'une des revendications précédentes, **caractérisé en ce que** la partie inférieure (44) d'au moins le premier système d'ancrage (44a), ou de chaque système d'ancrage (40a, 40b) comprend une partie annulaire (49) définissant un orifice central (50) de passage du premier élément de traction (30), avantageusement filiforme précité, de chaque élément de traction et pouvant aussi servir avantageusement au passage de l'aiguille (113) d'un trocart (110).

11. Dispositif selon l'une quelconque des revendications précédentes, **caractérisé en ce que** le premier élément allongé présente la forme d'une bandelette, dont au moins la partie entre ces extrémités est réalisée sous forme d'un tissu ou treillis formé à partir deux ou plusieurs fils réalisés en matériau souple, mais sensiblement non extensible.

12. Dispositif selon l'une quelconque des revendications précédentes, **caractérisé en ce que** le matériau utilisé pour la fabrication des fils servant à former le tissu de la bandelette est un polymère organique compatible avec une implantation à l'intérieur des tissus d'un mammifère, ce matériau organique étant avantageusement choisi parmi le polyéthylène ou le polypropylène, de préférence le polypropylène.

13. Dispositif selon l'une quelconque des revendications précédentes, **caractérisé en ce que** le deuxième élément de traction non extensible comprend ou est constitué d'un fil de traction non extensible, en particulier d'un fil réalisé en un matériau non extensible, par exemple un polymère organique compatible avec une implantation permanente dans les tissus d'un mammifère, en particulier en polypropylène, ou en nylon.

14. Elément de traction (30) du dispositif chirurgical formant prothèse chirurgicale (10) selon l'une quelconque des revendications 1 à 13, **caractérisé en ce que** ledit élément de traction (30) est réalisé en un matériau sensiblement non extensible, avantageusement filiforme, pouvant être rendu solidaire au moins provisoirement avec au moins une extrémité (22) ou (24) du premier élément de soutènement (20) allongé, et permet la traction et le maintien du premier élément de soutènement (20) allongé dans une position adaptée pour réaliser ledit soutien, tel que défini à l'une quelconque des revendications 1 à 13.

15. Système de fixation ou d'ancrage (40) du dispositif chirurgical formant prothèse chirurgicale (10) selon l'une quelconque des revendications 1 à 13, **caractérisé en ce que** ledit premier système d'ancrage (40) comprend un dispositif formant cage (42) comportant une extrémité supérieure (43) et une extrémité inférieure (44), l'extrémité supérieure (43) étant pourvue d'un élément de glissement (46) facilitant la traction de l'élément de traction (30) agissant sur l'élément de soutènement allongé (20), afin d'exercer par ladite traction un mouvement de translation d'un extrémité de l'élément de soutènement allongé (20) pour réaliser le soutènement et/ou le maintien dudit organe (200), tel que défini à l'une quelconque des revendications 1 à 13.

16. Kit de fixation et d'ancrage, **caractérisé en ce qu'**il comprend au moins un dispositif chirurgical formant prothèse chirurgicale (10), tel que défini à l'une quelconque des revendications 1 à 13, ainsi qu'un dispositif introducteur (110), avantageusement sous forme d'un trocart de pénétration (114), avec une gaine de protection (120) du système de fixation ou d'ancrage, qui est monté de manière compacte ou reliée entre le trocart de pénétration (110) et la gaine (120), et est solidaire de ladite gaine (120), au moins dans le sens de la pénétration (120), comportant un élément de blocage (121) anti-retrait du système de fixation en place entre le trocart (112, 114) et la gaine (120).

## Claims

1. A surgical device forming a surgical prosthesis (10), designed to provide support to a physiological, mammalian organ that can be supported, notably to correct urinary incontinence, comprising:
a) at least one first support component or sling (20) to provide support of elongated shape defining a first end (22) and a second end (24) designed to exert a supporting action on a physiological mammalian organ (200) requiring support; said first elongated component being made at least partially of a substantially inextensible but flexible and deformable material;
b) at least one first traction component (30), advantageously filiform, which can be connected at least temporarily with at least one end (22 or 24) of said first elongated sling (20), made of a substantially inextensible material which makes it possible to pull on the first elongated sling (20) and keep it in place in such a position that it can provide said support;
c) at least one first fixation or anchoring system (40), designed to cooperate with at least said first traction component (30), inextensible, in such a way as to fix and keep in the correct position said first elongated sling (20);
**characterized in that**
said first anchoring system (40) comprising a cage-forming device (42) with an upper end (43) and a lower end (44), the upper end (43) being fitted with a sliding component (46) which makes it easier to pull on the traction component (30) which acts on the elongated sling (20), in order to effect-through said pulling-the translation of one end of the elongated sling (20) in order to provide support to said organ (200) and/or to keep said organ in position.

2. Device according to Claim 1, **characterized in that** the lower end (44) of the anchoring system (40) is configured to be inserted on or attached to a mammalian substrate which is relatively resistant to tearing, e.g. bone or some other appropriate tissue.

3. Device according to Claim 1 or 2, **characterized in that** the upper part (43) and the lower part (44) of the cage-forming component (42) of the anchoring system (40) are joined to one another by a series of lateral bridging arms (48) made of a substantially inextensible but flexible and deformable material.

4. Device according to Claim 3, **characterized in that** said lateral bridging arms (48) are long enough to substantially form a kink at their mid-point (48a), in such a way that the upper part folds back on the lower part with the part of the arm (48b) joined to the lower part thus being also inserted on the above-mentioned mammalian substrate (150) which is relatively resistant to tearing, e.g. bone or some other appropriate tissue; the anchoring system is thus inserted over a broad area of mammalian substrate which is relatively resistant to tearing, which reduces or eliminates the risk of tearing thereof.

5. Device according to any of claims 1 to 4, **characterized in that** the cage-shaped sliding component (46) of the anchoring system (40) is configured so as to act like a pulley, around which the traction component (30) can slide or translate to pull on the elongated sling (20).

6. Device according to any of the preceding claims, **characterized in that** the traction component (30) is in the form of a traction wire, preferably including a sliding component (32) such as a ring or a loop at one end (31), the other end of the wire-the free end-can be inserted into this ring or loop in such a way as to create a lasso-like loop device to facilitate pulling on the elongated sling (20); preferably, the elongated sling (20) contains at least one hole (26, 27) through which said traction wire (30) can be passed to be trapped by the lasso-type device which, when the other end of the wire (33)-the free end-is pulled, effects translation of said end (22 or 24) of the elongated sling (20), e.g. a strip.

7. Device according to any of the preceding claims, **characterized in that** a unique anchoring system is provided to simultaneously pull both ends (22 and 24) of the sling (20), e.g. a strip.

8. Device according to any of the preceding claims, **characterized in that** a first anchoring system (40a) joined to a first end (22) of the elongated sling (e.g. a strip) is configured to allow fixation at a predetermined, set position, while the other end (24) of the sling (20) is joined to a second anchoring system (40b) according to the present invention, thereby allowing displacement of the second end (24) of the sling (20) in order to exert a traction force on the mammalian organ as required.

9. Device according to any of the preceding claims, **characterized in that** the cage-forming device (42) includes at its upper end (43) a hollow, tubular component (47), one end of which (47a) is designed to be joined to or continuous with the upper part (43) of the cage (42) and, at the same time, to form the sliding component (46), in particular by presenting a surface which forms the pulley around which the traction wire (30) is designed to slide; advantageously, said end (47a) has a hole (60) which is coaxial with the hollow tubular component (47).

10. Device according to any of the preceding claims, **characterized in that** the lower part (44) of at least the first anchoring system (44a) or of each anchoring system (40a and 40b) includes an annular part (49) defining a central hole (50) through which can be passed the first traction component (30)-advantageously filiform as mentioned previously-or of each traction component, and which can also be advantageously used to accommodate the needle (113) of a trocar (110).

11. Device according to any of the preceding claims, **characterized in that** the first elongated sling is in the shape of a strip, of which at least that part which is located between its ends consists of a fabric or meshwork created from two or more wires made of a substantially inextensible but flexible material.

12. Device according to any of the preceding Claims, **characterized in that** the material used to make the wires comprising the fabric of the strip is an organic polymer which is compatible with implantation in mammalian tissue, this organic material being advantageously one of the group of polyethylene, polypropylene or nylon and preferably polypropylene.

13. Device according to any of the preceding Claims, **characterized in that** the second inextensible traction component includes or is constituted of an inextensible traction wire, in particular a wire made of an inextensible material, e.g. an organic polymer which is compatible with long-term implantation in mammalian tissue, in particular polypropylene or nylon.

14. Traction component (30) of the surgical device forming a surgical prosthesis (10) according to any of claims 1 to 13, **characterized in that** said traction component (30) is made of a substantially inextensible material, advantageously filiform, and can be joined at least temporarily with at least one end (22 or 24) of the first elongated sling (20), to allow the pulling of said elongated sling (20) and keep said elongated sling (20) in a position where it can provide said support, as defined in any of claims 1 to 13.

15. Fixation or anchoring system (40) of the surgical device forming a surgical prosthesis (10) according to any of claims 1 to 13, **characterized in that** said anchoring system (40) comprises a cage-forming device (42) with an upper end (43) and a lower end (44), the upper end (43) being fitted with a sliding component (46) which makes it easier to pull on the traction component (30) which acts on the elongated sling (20), in order to effect-through said pulling-the translation of one end of the elongated sling (20) in order to provide support to said organ (200) and/or to keep said organ in position, as defined in any of claims 1 to 13.

16. Fixation and anchoring kit **characterized in that** it comprises at least one surgical device forming a surgical prosthesis (10), as defined in any of claims 1 to 13, together with an introducer instrument (110), advantageously in the form of a penetrating trocar (114), with a protective sheath (120) for the fixation or anchoring system mounted in a compact way or folded back between the penetrating trocar (110) and the sheath (120), and joined to said sheath (120) at least in the direction of penetration, said sheath including a system (121) to prevent retrocession of the fixation system in place between the trocar (112, 114) and the sheath (120).

## Patentansprüche

1. Chirurgische Vorrichtung, die eine chirurgische Prothese (10) bildet, welche zum Stützen eines zu unterstützenden biologischen Organs eines Säugetiers bestimmt ist, insbesondere zur Behandlung von Harninkontinenz, umfassend:
a) mindestens ein erstes Stützelement (20) in lang gestreckter Form, das ein erstes (22) und ein zweites (24) Ende definiert, um eine Stützwirkung für ein zu unterstützendes biologisches Organ (200) eines Säugetieres auszuüben; wobei das erste Element in lang gestreckter Form mindestens teilweise aus einem im wesentlichen nicht dehnbaren, flexiblen, verformbaren Material verwirklicht ist;
b) mindestens ein erstes Zugelement (30), das vorteilhafterweise fadenförmig ist, wobei es mindestens provisorisch mit mindestens einem Ende (22) oder (24) des ersten lang gestreckten Zugelements (20) verbunden werden kann, das aus einem im Wesentlichen nicht dehnbaren Material verwirklicht ist und das den Zug und das Halten des ersten lang gestreckten Stützelements (20) in einer angepaßten Position zur Durchführung des Stützens ermöglicht;
c) mindestens ein erstes Befestigungs- oder Verankerungssystem (40), das zur Zusammenarbeit mit mindestens dem ersten nicht dehnbaren Zugelement (30) bestimmt ist, sodaß das Befestigen und Halten in der angemessenen Position des ersten lang gestreckten Stützelements (20) ermöglicht wird;
**dadurch gekennzeichnet, daß**
das erste Verankerungssystem (40), das eine Vorrichtung, die ein Gehäuse (42) bildet, aufweist, ein oberes Ende (43) und ein unteres Ende (44) umfaßt, wobei das obere Ende (43) mit einem Gleitelement (46) versehen ist, das den Zug des Zugelements (30) erleichtert, das auf das lang gestreckte Stützelement (20) wirkt, um durch diesen Zug eine Übersetzungsbewegung von einem Ende des lang gestreckten Stützelements (20) auszuüben, um das Stützen und/oder Halten des Organs (200) zu verwirklichen.

2. Vorrichtung nach Anspruch 1, **dadurch gekennzeichnet, daß** das untere Ende (44) des Verankerungssystems (40) angepaßt ist, um auf einem Träger (150) aufzuliegen oder sich auf diesem befestigen zu lassen, der gegenüber dem Reißen des Säugetiers, wie etwa dementsprechende Knochen oder Gewebe, relativ widerstandsfähig ist.

3. Vorrichtung nach Anspruch 1 oder 2, **dadurch gekennzeichnet, daß** der obere Teil (43) und der untere Teil (44) des Elements, das das Gehäuse (42) des Verankerungssystems (40) bildet, miteinander durch mehrere seitliche Verbindungsarme (48) verbunden sind, die aus einem im Wesentlichen nicht dehnbaren aber flexiblen, verformbaren Material verwirklicht sind.

4. Vorrichtung nach Anspruch 3, **dadurch gekennzeichnet, daß** die seitlichen Verbindungsarme (48) eine ausreichende Länge aufweisen, um im Wesentlichen in ihrer Mitte (48a) einen Faltpunkt der seitlichen Arme (48) zu bilden, sodaß der obere Teil in Richtung des unteren Teils umgefaltet wird, wobei sich der Teil der Arme (48b), der mit dem unteren Teil verbunden ist, somit ebenfalls auf den vorher erwähnten Träger (150) zu liegen kommt, welcher gegenüber dem Reißen der Säugetiere, wie etwa dementsprechende Knochen oder Gewebe, relativ widerstandsfähig ist; wobei das Verankerungssystem somit auf dem Träger zu liegen kommt, welcher gegenüber dem Reißen der Säugetiere auf einer großen Fläche relativ widerstandsfähig ist, was sein Reißen einschränkt oder verhindert.

5. Vorrichtung nach einem der Ansprüche 1 bis 4, **dadurch gekennzeichnet, daß** das Gleitelement (46) des Verankerungssystems (40) in Form eines Gehäuses derart konzipiert ist, um wie eine Riemenscheibe zu wirken, um die das Zugelement (30) gleitet, um auf das lang gestreckte Stützelement (20) die Zugwirkung auszuüben.

6. Vorrichtung nach einem der vorhergehenden Ansprüche, **dadurch gekennzeichnet, daß** das Zugelement (30) die Form eines Zugfadens aufweist, der vorzugsweise an einem Ende (31) ein Gleitelement (32), wie etwa ein Ring oder eine Schleife, umfaßt, in den/die das andere Ende oder der freie Teil (33) des Fadens eingeführt werden kann, um so die Bildung der Vorrichtung als Schlaufe der Art Lasso zu ermöglichen, die einen erleichterten Zug auf das lang gestreckte Stützelement (20) ermöglicht; vorzugsweise weist das lang gestreckte Stützelement (20) mindestens eine Öffnung (26, 27) auf, durch die der Zugfaden (30) verlaufen kann, um somit in der Vorrichtung der Art Lasso gefangen zu werden, die durch einen Zug, der auf das andere Ende oder den freien Teil (33) des Fadens ausgeübt wird, die Übersetzung durch den Zug des Endes (22) oder (24) des lang gestreckten Stützelementes (20), zum Beispiel ein Bändchen, verwirklicht.

7. Vorrichtung nach einem der vorhergehenden Ansprüche, **dadurch gekennzeichnet, daß** ein einzigartiges Verankerungssystem vorgesehen ist, um gleichzeitig jedes Ende (22, 24) des lang gestreckten Stützelementes (20), zum Beispiel ein Bändchen, zu ziehen.

8. Vorrichtung nach einem der vorhergehenden Ansprüche, **dadurch gekennzeichnet, daß** ein erstes Verankerungssystem (40a), das mit einem ersten Ende (22) des lang gestreckten Stützelementes (20), zum Beispiel ein Bändchen, verbunden ist, vorgesehen ist, um eine Befestigung in einer vorherbestimmten festen Position zu ermöglichen, wohingegen das andere Ende (24) des lang gestreckten Stützelementes (20), zum Beispiel ein Bändchen, mit einem zweiten Verankerungssystem (40b) verbunden wird, wobei das Ausüben einer Verschiebung des zweiten Endes (24) des lang gestreckten Stützelementes (20) ermöglicht wird, um die gesuchte Zugwirkung auf das Organ (200) des Säugetieres, welches diese braucht, auszuüben.

9. Vorrichtung nach einem der vorhergehenden Ansprüche, **dadurch gekennzeichnet, daß** die Vorrichtung, die ein Gehäuse (42) bildet, an ihrem oberen Ende (43) ein hohles Rohrelement (47) umfaßt, wovon ein Ende (47a) derart konzipiert ist, um zugleich mit dem oberen Teil (43) des Gehäuses (42) verbindbar oder in einem Stück zu sein und das Gleitelement (46) zu bilden, insbesondere, indem es eine Oberfläche aufweist, die eine Riemenscheibe bildet, um die der Zugfaden (30) gleiten soll; vorteilhafterweise umfaßt dieses Ende (47a) eine Durchführungsöffnung (60), die mit dem hohlen Rohrelement (47) koaxial ist.

10. Vorrichtung nach einem der vorhergehenden Ansprüche, **dadurch gekennzeichnet, daß** der untere Teil (44) von mindestens dem ersten Verankerungssystem (44a) oder von jedem Verankerungssystem (40a, 40b) einen ringförmigen Teil (49) umfaßt, der eine zentrale Durchgangsöffnung (50) des ersten Zugelementes (30), das vorteilhafterweise wie oben erwähnt fadenförmig ist, jedes Zugelementes definiert und ebenfalls vorteilhafterweise zum Durchgang der Nadel (113) eines Trokars (110) dienen kann.

11. Vorrichtung nach einem der vorhergehenden Ansprüche, **dadurch gekennzeichnet, daß** das erste lang gestreckte Element die Form eines Bändchens aufweist, wovon mindestens der Teil zwischen den Enden in Form eines Gewebes oder Geflechtes, das aus zwei oder mehreren Fäden gebildet ist, verwirklicht ist, die aus einem flexiblen aber im Wesentlichen nicht dehnbaren Material verwirklicht sind.

12. Vorrichtung nach einem der vorhergehenden Ansprüche, **dadurch gekennzeichnet, daß** das Material, das zur Herstellung der Fäden, die zum Bilden des Gewebes des Bändchens dienen, benutzt wird, ein organisches Polymer ist, das mit einem Implantat im Inneren der Gewebe eines Säugetieres kompatibel ist, wobei dieses organische Material vorteilhafterweise aus Polyethylen oder Polypropylen, vorzugsweise Polypropylen, ausgewählt ist.

13. Vorrichtung nach einem der vorhergehenden Ansprüche, **dadurch gekennzeichnet, daß** das zweite nicht dehnbare Zugelement einen nicht dehnbaren Zugfaden aufweist oder aus diesem zusammengesetzt ist, insbesondere aus einem Faden, der aus einem nicht dehnbaren Material verwirklicht ist, zum Beispiel einem organischen Polymer, das mit einem permanenten Implantat in den Geweben eines Säugetieres kompatibel ist, insbesondere aus Polypropylen oder Nylon.

14. Zugelement (30) der chirurgischen Vorrichtung, die die chirurgische Prothese (10) nach einem der Ansprüche 1 bis 13 bildet, **dadurch gekennzeichnet, daß** das Zugelement (30) aus einem im Wesentlichen nicht dehnbaren, vorteilhafterweise fadenförmigen Material verwirklicht ist, das mindestens provisorisch mit mindestens einem Ende (22) oder (24) des ersten lang gestreckten ersten Stützelementes (20) verbunden werden kann und den Zug und das Halten des ersten lang gestreckten Stützelementes (20) in einer an die Verwirklichung dieser Stütze angepaßten Position, wie in einem der Ansprüche 1 bis 13 definiert, ermöglicht.

15. Befestigungs- oder Verankerungssystem (40) der chirurgischen Vorrichtung, die die chirurgische Prothese (10) nach einem der Ansprüche 1 bis 13 bildet, **dadurch gekennzeichnet, daß** das erste Verankerungssystem (40) eine ein Gehäuse (42) bildende Vorrichtung aufweist, die ein oberes Ende (43) und ein unteres Ende (44) umfaßt, wobei das obere Ende (43) mit einem Gleitelement (46) versehen ist, das den Zug des Zugelementes (30), das auf das lang gestreckte Stützelement (20) wirkt, erleichtert, um durch diesen Zug eine Übersetzungsbewegung eines Endes des lang gestreckten Stützelementes (20) auszuüben, um das Stützen und/oder Halten des Organs (200), wie in einem der Ansprüche 1 bis 13 definiert, zu verwirklichen.

16. Befestigungs- und Verankerungssatz, **dadurch gekennzeichnet, daß** er mindestens eine chirurgische Vorrichtung aufweist, die die chirurgische Prothese (10), wie in einem der Ansprüche 1 bis 13 definiert, bildet, sowie eine Einführungsvorrichtung (110), vorteilhafterweise in Form eines Durchdringungstrokars (114) mit einer Schutzhaube (120) des Befestigungs- oder Verankerungssystems, die auf kompakte Weise montiert oder zwischen dem Durchdringungstrokar (110) und der Haube (120) verbunden ist, und mit dieser Haube (120) mindestens in der Richtung der Durchdringung (120) verbunden ist, die ein Anti-Rückzugs-Blockierungselement (121) des Befestigungssystems an der Stelle zwischen dem Trokar (112, 114) und der Haube (120) umfaßt.
